# EUROPÄISCHE PATENTSCHRIFT

(11) **EP 1 008 350 B1**
(45) Veröffentlichungstag und Bekanntmachung des Hinweises auf die Patenterteilung: **16.11.2005**
(21) Anmeldenummer: 99122673.9
(22) Anmeldetag: 15.11.1999
(51) Int. Cl.: A61K 35/16

(54) **Stabilisiertes Antithrombin III-Präparat**
Stabilised antithrombin III preparation
Préparation stabilisée de l'antithrombine III

(30) Priorität: 08.12.1998 DE 19856443
(43) Veröffentlichungstag der Anmeldung: 14.06.2000
(73) Patentinhaber: ZLB Behring GmbH, 35041 Marburg (DE)
(72) Erfinder: Römisch, Jürgen Dr., 35041 Marburg (DE); Stauss, Harald, 35232 Dautphetal (DE)

(56) Entgegenhaltungen:
- EP-A- 0 018 561
- US-A- 4 340 589
- US-A- 4 623 717
- BUSBY T F ET AL: "THERMAL STABILIZATION OF ANTITHROMBIN III BY SUGARS AND SUGAR DERIVATIVES AND THE EFFECTS OF NONENZYMATIC GLYCOSYLATION" BIOCHIMICA ET BIOPHYSICA ACTA,NL,AMSTERDAM, Bd. 799, Nr. 1, 25. Mai 1984 (1984-05-25), Seiten 80-89, XP000674342 ISSN: 0006-3002

## Beschreibung

Gegenstand der Erfindung ist ein stabilisiertes Antithrombin III-Präparat, das durch den Zusatz von Stabilisatoren gegen einen Wirkungsverlust bei der Pasteurisierung geschützt ist sowie ein Verfahren zur Virusinaktivierung eines derartigen Antithrombin III-Präparats.

Antithrombin III (ATIII) ist einer der wichtigsten plasmatischen Inhibitoren. ATIII gehört zu der Familie der Serinprotease-Inhibitoren, die mit ihren "Zielproteasen" einen der kovalenten Bindung nahe kommenden Komplex eingehen. Dieser Komplex ist unter physiologischen Bedingungen sehr stabil und wird in der Regel schnell aus dem Blutkreislauf eliminiert. Die Reaktion zwischen ATIII und der Protease wird durch Heparin drastisch beschleunigt, wobei das ATIII nach Assoziation mit dem Glykosaminoglykan eine leichte Konformationsänderung erfährt und damit eine beschleunigte Reaktion mit der Protease eingehen kann. Diese Vorgänge spielen physiologisch besonders an Zelloberflächen eine Rolle, die Glykosaminoglykane z.B. vom Typ des Heparansulfates enthalten und somit eine Barriere der Zellen und Gewebe vor überhöhter proteolytischer Aktivität darstellen. Daneben kommt aber auch der plasmatischen Gerinnung und deren Regulation eine wichtige Bedeutung zu.

Besonders deutlich wird die regulatorische Funktion dieses Inhibitors, wenn die ATIII Plasmaspiegel sinken, wie es bei vielen Krankheiten und besonders drastisch z.B. im Falle einer disseminierten intravasalen Gerinnung (DIC) beobachtet wird. Bereits ein Unterschreiten von 70% der entsprechenden Plasmakonzentration ist mit einer drastischen Erhöhung der Mortalitätswahrscheinlichkeit verbunden. Ein Überwiegen von Gerinnungsprozessen führt häufig zu thrombotischen Verschlüssen von Gefäßen und damit zum Organversagen. Entsprechend hat sich die Applikation von ATIII-Konzentraten aus humanem Plasma besonders in Fällen von angeborenen and erworbenen Mangelzuständen als sehr hilfreich erwiesen.

Patienten, die an einem angeborenen oder erworbenen ATIII-Mangel leiden, werden derzeit durch Substitution von aus Humanplasma gewonnenen Konzentraten therapiert. Neben der Effektivität dieser Konzentrate muß besonders die Sicherheit hinsichtlich des potentiellen Risikos einer Übertragung von Infektionskrankheiten gewährleistet sein. Neben Virusinaktivierungsverfahren, wie der Behandlung mit Detergenzien z.B. nach der SD (solvent/detergent)-Methode kommt dafür auch die als Pasteurisierung bekannte Hitze-Inaktivierung von Viren bei 60°C in Betracht, die bereits in den 40er Jahren für Albumin angewendet wurde. Im Allgemeinen werden bei einer Pasteurisierung Proteine bis zu 10 Stunden bei 60°C behandelt. Diese hohe Temperatur kann aber zu Denaturierungen der Proteine führen, die Verluste der Wirksamkeit und der Ausbeute zur Folge haben. Beim ATIII spiegelt sich dies in dem Verlust der heparinbindenden Eigenschaften eines bestimmten Proteinanteiles wider. Dieser ATIII-Anteil ist nicht mehr in einem Heparin-Kofaktortest meßbar und zeigt sich in der zweidimensionalen Immunelektrophorese als separater Peak, der eindeutig von dem Heparin-bindenden Anteil unterscheidbar ist.

Den meist auf konformationellen Veränderungen der Proteine zurückzuführenden Veränderungen wird durch Zugabe von Stabilisatoren zu der zu erhitzenden Lösung entgegengewirkt. Zur Erzielung eines optimalen Schutzes derartiger Proteine müssen die hierfür eingesetzten Stabilisatoren sowohl in ihrer Zusammensetzung als auch in den mengenmäßigen Anteilen der einzelnen Bestandteile des Stabilisatorgemisches genau auf jedes Protein abgestimmt sein. Dementsprechend ist aus der US-Patentschrift 4 297 344 ein spezielles Stabilisatorgemisch für ATIII bekannt, das bei Pasteurisierungsverfahren angewendet wird. Dabei wird ATIII in wäßriger Lösung mit einem Kohlenhydrat wie Saccharose und mindestens einer Aminosäure aus der Reihe Glycin, α- und β-Alanin, Hydroxy-Prolin, Glutamin und α- , β- oder γ-Buttersäure versetzt. Damit läßt sich jedoch keine vollständig befriedigende Stabilisierung von ATIII erreichen, da nach der Pasteurisierung der Heparin nicht- bindende Anteil über 12% liegt.

Es wurde nun gefunden, daß ATIII gegen einen Wirkungsverlust bei der Pasteurisierung wesentlich effektiver geschützt werden kann, wenn als Stabilisatoren entweder ein oder mehrere Saccharide in erhöhter Konzentration (> 1 g/ml) oder ein oder mehrere Saccharide in Kombination mit einer oder mehreren Aminosäuren aus der Gruppe Arginin, Lysin, Histidin, Phenylalanin, Tryptophan, Tyrosin, Asparaginsäure und ihren Salzen sowie Glutaminsäure und ihren Salzen ausgewählt werden. Dabei kann eine oder mehrere dieser Aminosäuren auch mit Glycin und/oder Glutamin kombiniert werden.

Die Stabilisatoren werden normalerweise in einer Pufferlösung, z.B. einer Citratlösung, eingesetzt.

Als Saccharid kann ein Monosaccharid, ein Disaccharid oder ein Oligosaccharid in einer Menge von wenigstens 0,5 g/ml eingesetzt werden. Bevorzugt ist jedoch ein Wert über 1,0 g/ml, wobei gleichzeitig pH-Werte von 6,0 bis 9,5, bevorzugt von 7,0 bis 8,5 angewendet werden.

Die vorstehend genannten Aminosäuren werden allein oder in Kombination in einer Menge von wenigstens 0,1 mol/l, vorzugsweise jedoch in einer Menge von mehr als 0,5 mol/l eingesetzt. Besonders bevorzugt werden dabei Kombinationen von einem oder mehreren Zuckern in einer Konzentration von mehr als 1,5 g/ml und einer oder mehreren Aminosäuren in Konzentrationen von jeweils mehr als 0,1 mol/l. Ebenfalls besonders bevorzugt ist die Kombination von einem Zucker in einer Menge von mehr als 1,5 g/ml und einer oder mehreren der obengenannten Aminosäuren mit Glycin und/oder Glutamin, die alle jeweils in Konzentrationen von über 0,2 mol/l zur Anwendung kommen Als Saccharid kann in der Stabilisatormischung ein Monosaccharid, ein Disaccharid oder ein Oligosaccharid in einer Menge von über 1,0 g/ml, wobei gleichzeitig pH-Werte von 6,0 bis 9,5, bevorzugt von 7,0 bis 8,5 angewendet werden.

Die vorstehend genannten Aminosäuren werden zur Stabilisierung in einer Menge von mehr als 0,5 mol/l eingesetzt. Besonders bevorzugt werden dabei Mischungen von einem oder mehreren Zuckern in einer Konzentration von mehr als 1,5 g/ml in Mischungen mit einer oder mehreren Aminosäuren, die in Konzentrationen von mehr als 0,5 mol/l angewendet werden. Ebenfalls besonders bevorzugt ist die Kombination von einem Zucker in einer Menge von mehr als 1,5 g/ml und einer oder mehreren der obengenannten Aminosäuren mit Glycin und/oder Glutamin, die in Konzentrationen von über 0,5 mol/l zur Anwendung kommen sollten.

Die so stabilisierte Lösung des ATIII wird zur Virusinaktivierung 5 bis 50 Stunden, bevorzugt 8 bis 20 Stunden, bei 40 bis 95°C, bevorzugt bei 50 bis 70°C erhitzt. Am günstigsten ist jedoch eine Virusinaktivierung bei 55 bis 65°C.

Die Herstellungsverfahren für das ATIII-Konzentrat, wie chromatographische Verfahren mittels immobilisierten Heparins sind dem Fachmann bekannt. Das erfindungsgemäße Verfahren zur Virusinaktivierung wird im allgemeinen auf aus Plasma gewonnenes ATIII angewendet und ist unabhängig von der Plasmafraktion, die als Ausgangsprodukt für die weitere Reinigung des ATIII gewählt wird. Dies gilt für beide Isoformen des ATIII, das sogenannte ATIII-alpha als auch das ATIII-beta. Die Erfindung kann ebenso auch auf rekombinant hergestelltes oder transgenes ATIII angewendet werden.

Die beschriebene Stabilisierung kann auch bei anderen Virusinaktivierungsverfahren angewendet werden.

### Beispiel 1

Jeweils 5 bis 10 ml einer Lösung, die ATIII in einer Konzentration von etwa 200 IU/ml enthielt, wurden mit Saccharose allein und in Kombination mit einer oder mehreren Aminosäuren versetzt und für 10 Stunden bei 60°C inkubiert. Anschließend wurde die Nativität des ATIII im Sinne der Heparin-bindenden Eigenschaften durch zweidimensionale Immunelektrophorese bestimmt. Dazu wurde eine Elektrophorese der Proteinlösung in Gegenwart von Heparin durchgeführt. Die Heparin-bindenden Moleküle wurden dadurch stärker negativ geladen und wanderten entsprechend schneller im elektrischen Feld. Danach wurde ein Agarosegel unter Zugabe eines polyklonalen Antikörpers gegen ATIII an das erste Gel angegossen und das elektrische Feld im rechten Winkel zur ersten Laufrichtung angelegt. In Bereichen äquimolarer Verhältnisse von ATIII zu Antikörper fand eine Präzipitationsreaktion statt. Nach Wässern des Geles wurde die Präzipitationslinie in Form einer Kurve bzw. eines Peaks durch Anfärbung z.B. mit Coomassie Blue deutlicher sichtbar gemacht. Eine Quantifizierung des Heparin-bindenden bzw nicht-bindenden Anteiles erfolgte nach Ziehen der Basislinie mit Hilfe eines Scanners und der Integration der Peakflächen, deren Summe gleich 100% gesetzt wurde. Die entsprechenden Peaks wurden dazu in das Verhältnis gesetzt und der entsprechende Anteil in Prozent ausgedruckt. Die Nachweisgrenze dieser Methode liegt bei 3% Heparin-nicht-bindendem Anteil.

Es wurden jeweils mehrere Lots getestet, wobei immer die ATIII Lösung vor Pasteurisierung als Kontrolle eingeschlossen wurde.

Folgende Ansätze wurden pasteurisiert und wie beschrieben ausgewertet. Die Ergebnisse sind als Mittelwerte von 2 bis 5 Lots dargestellt. Die Versuche Nr. 1, 2, 3A und 3B stellen den Stand der Technik dar. Die Versuche 4, 5, 6, 7 und 8 zeigen, daß bei einem erfindungsgemäß stabilisierten Antithrombin III-Präparat der Heparin-nicht-bindende Anteil zwischen etwa 3 und 4% liegt.

| **Nr.** | **Saccharose (g/ml)** | **Aminosäuren (mol/l)** | **Heparin nicht bindender Anteil (%)** |
|---|---|---|---|
| 1 | vor Pasteurisierung | | <3 |
| 2 | 0,5 | --- | >20 |
| 3A | 1,0 | Glycin (2 mol/l) | 12,8 |
| 3B | 1,0 | Glycin (2 mol/l) (Arginin (2 mol/l)/Glutamat (2 mol/l) wurden erst nach der Pasteurisierung zugesetzt) | 12,6 |
| 4 | 1,75 | Glycin (2 mol/l)/Glutamat (2 mol/l) | < 3,5 |
| 5 | 1,75 | Glycin (2 mol/l)/Glutamat (2 mol/l) | < 3 |
| 6 | 1,75 | Arginin(2mol/l) | 4,3 |
| 7 | 1,75 | Lysin (2 mol/l) | 3,9 |
| 8 | 1,75 | Glutamat (1 und 2 mol/l) | < 3 |

### Ergebnis:

Der jeweilige Ansatz vor Pasteurisierung (Nr. 1) enthielt erwartungsgemäß in jedem Fall <3% Heparin nicht-bindenden Anteil ATIII. Ansätze ohne bzw. sehr geringe Stabili-satormengen zeigten nach Erhitzung >20% des nicht-bindenden Proteins (Nr. 2).

Die Mischungen mit Glycin/Glutamat (Nr. 4) mit im Mittel <3,5% und besonders mit Glycin/Glutamat/Arginin (Nr. 5) mit <3% zeigten sehr effektive stabilisierende Wirkungen. Auch die Kombinationen von Saccharose z.B. mit Arginin oder Lysin (Nr. 6,7) haben sich als vorteilhaft herausgestellt. Bereits Glutamat allein bewirkte eine sehr effektive Stabilisierung (Nr. 8).

Zur Untersuchung, ob die zugesetzten Stabilisatoren, besonders die Aminosäuren Glutamat und Arginin, allein das elektro-phoretische Laufverhalten beeinflußten, wurden diese (Nr. 3B) erst nach der Pasteurisierung dem Gemisch Saccharose/Glycin zugesetzt und mit der Kontrolle (3A) verglichen. Es wurde demonstriert, daß diese Zusätze keinen signifikanten Einfluß auf die Elektrophorese hatten.

### Beispiel 2:

Die Abhängigkeit der Stabilisierung von der Zuckerkonzentration wurde entsprechend der Versuchsdurchführung in Beispiel 1 untersucht.

| **Nr.** | **Saccharose (g/m1)** | **Aminosäuren (mol/l)** | **Heparin nichtbindender Anteil (%)** |
|---|---|---|---|
| 1 | vor Pasteurisierung | | < 3 |
| 2 | 0,5 | --- | >20 |
| 3A | 1,0 | Glycin (2 mol/l)/Glutamat | 6,8 |
| 3B | 1,0 | Glycin (2 mol/l) | 12,8 |
| 3C | 1,75 | Glycin (2 mol/l) | 5,4 |
| 4A | 0,5 | Glutamat (1 mol/l)/Arginin (2 mol/l) | 14,1 |
| 4B | 1,0 | Glutamat (1 mol/l)/Arginin (2 mol/l) | 6,4 |
| 4C | 1,75 | Glutamat (1 mol/l)/Arginin (2 mol/l) | < 3 |

### Ergebnis:

Sowohl die Ansätze 3B und C als auch 4A bis C zeigen, daß die Erhöung des Zuckeranteiles einen wichtigen Beitrag zur Stabilisierung lieferte. Der zusätzlich stabiliserende Effekt von Glutamat/Arginin (4B) oder Glutamat (3A) bzw. gegenüber den Ansätzen mit Glycin (3A versus 3B) bei einer Saccharose-Konzentration von lediglich 1 mol/l wurde ebenfalls deutlich.

## Patentansprüche

1. Stabilisiertes flüssiges Antithrombin III-Präparat (AT III), das durch den Zusatz von Stabilisatoren gegen einen Wirkungsverlust bei der Pasteurisierung geschützt ist, wobei als Stabilisatoren eingesetzt werden
a) ein oder mehrere Saccharide in einer Konzentration von über 1 g/ml, und
b) eine oder mehrere Aminosäuren, in einer Konzentration von mehr als 0,5 mol/l, ausgewählt aus der Gruppe
Arginin, Lysin, Histidin, Phenylalanin, Tryptophan, Tyrosin, Asparaginsäure und ihren Salzen, Glutamin oder Glutaminsäure und ihren Salzen.

2. Antithrombin III-Präparat nach Anspruch 1, **dadurch gekennzeichnet, daß** zusätzlich Glycin zugesetzt ist.

3. Antithrombin III-Präparat nach Anspruch 1 oder 2, **dadurch gekennzeichnet, dass** es zusätzlich bis zu 15 % (Gew/ Vol) Ammoniumsulfat enthält.

4. Verfahren zur Virusinaktivierung eines Antithrombin III-Präparates, **dadurch gekennzeichnet, daß** man ein Antithrombin III-Präparat gemäß den Ansprüchen 1 bis 3 einer Hitzebehandlung bei 40 bis 95°C über einen Zeitraum von 5 bis 50 Stunden unterwirft.

## Claims

1. A stabilized liquid antithrombin III preparation (AT III) which is protected against a loss of action during pasteurization by the addition of stabilizers, use being made as stabilizers of
a) one or more saccharides in a concentration of more than 1g/ml and
b) one or more amino acids in a concentration of more than 0.5 mol/l chosen from the group consisting of arginine, lysine, histidine, phenylalanine, tryptophan, tyrosine, aspartic acid and its salts, glutamine or glutamic acid and its salts.

2. The antithrombin III preparation as claimed in claim 1, wherein additionally glycine has been added.

3. The antithrombin III preparation as claimed in claim 1 or 2, which additionally contains up to 15% (w/v) of ammonium sulfate.

4. A process for virus inactivation of an antithrombin III preparation, which comprises subjecting an antithrombin III preparation as claimed in claims 1 to 3, to heat treatment at 40 to 95°C over a period of time of 5 to 50 hours.

## Revendications

1. Préparation liquide stabilisée d'antithrombine III (AT III), qui est protégée par addition d'agents stabilisants contre une perte d'activité lors de la pasteurisation, dans laquelle on utilise comme agents stabilisants
a) un ou plusieurs saccharides à une concentration supérieure à 1 g/ml, et
b) un ou plusieurs acides aminés, à une concentration supérieure à 0,5 mole/l, choisis dans le groupe
arginine, lysine, histidine, phénylalanine, tryptophane, tyrosine, acide asparagique et leurs sels, glutamine ou acide glutamique et leurs sels.

2. Préparation d'antithrombine III selon la revendication 1, **caractérisée en ce que** de la glycine est ajoutée en supplément.

3. Préparation d'antithrombine III selon la revendication 1 ou 2, **caractérisée en ce qu'**elle contient additionnellement jusqu'à 15% (poids/volume) de sulfate d'ammonium.

4. Procédé d'inactivation des virus d'une préparation d'antithrombine III, **caractérisé en ce qu'**on soumet une préparation d'antithrombine III selon les revendications 1 à 3 à un traitement par la chaleur de 40 à 95°C pendant une durée de 5 à 50 heures.
